(19) Europäisches Patentamt European Patent Office Office européen des brevets

(11) **EP 4 785 923 A1**

(12) **EUROPEAN PATENT APPLICATION**
published in accordance with Art. 153(4) EPC

(43) Date of publication:
05.08.2026 Bulletin 2026/32

(21) Application number: 24872332.2

(22) Date of filing: 25.09.2024

(51) International Patent Classification (IPC):
*A61K 6/76* (2020.01)    *A61K 6/17* (2020.01)
*C01B 33/18* (2006.01)

(52) Cooperative Patent Classification (CPC):
A61K 6/17; A61K 6/76; C01B 33/18

(86) International application number:
PCT/JP2024/034288

(87) International publication number:
WO 2025/070546 (03.04.2025 Gazette 2025/14)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 27.09.2023 PCT/JP2023/035047

(71) Applicant: ADMATECHS CO., LTD.
Miyoshi-shi, Aichi, 470-0201 (JP)

(72) Inventors:
• OOSAWA Kazuma
Miyoshi-shi, Aichi 470-0201 (JP)
• HORI Kenta
Miyoshi-shi, Aichi 470-0201 (JP)
• NAKAMURA Tempo
Miyoshi-shi, Aichi 470-0201 (JP)

(74) Representative: Winter, Brandl - Partnerschaft
mbB
Alois-Steinecker-Straße 22
85354 Freising (DE)

(54) **PARTICLE MATERIAL AND DENTAL MATERIAL**

(57) An object is to provide a particulate material used as a filler for a dental material, and a dental material using the particulate material. A particulate material of the present disclosure that achieves the above-described object is formed of an aggregate including: an interior composed of primary particles including two or more types of metal elements, at least one of the metal elements being a heavy element having an atomic number of 38 or greater in the form of a metal oxide, particles of the metal oxide of the heavy element being composed of the primary particles having a volume-average particle diameter of 1 nm to 20 nm; and a surface composed of silica, the surface including primary particles composed of silica and having a volume-average particle diameter of 1 nm to 20 nm. The aggregate has a specific surface area of 180 m$^2$/g or less, a linseed oil absorption amount per unit volume (100 cm$^3$) of 200 g or less, a linseed oil absorption amount per unit surface area (m$^2$) of 0.02 g or less, and a light transmittance of more than 10%, and a mass of the heavy element is 4% to 60% based on a total mass. The use of the aggregate makes it possible to enhance strength while achieving optical performance comparable to that of materials having a small particle diameter.

FIG.1

SU8000 2.0kV 8.0mm x10.0k SE(U)                    5.00um

EP 4 785 923 A1

**Description**

Technical Field

The present disclosure relates to a particulate material and a dental material using the particulate material.

Background Art

As conventional dental materials, resin compositions commonly referred to as composite resins, in which inorganic particulate materials are dispersed in a resin material, are generally used (e.g., Patent Literature 1). As the resin material, a material in a pre-cured state such as a monomer is used. After the composite resin is filled into a defective portion of a tooth, the resin material undergoes polymerization and curing when external energy such as light irradiation is applied. The present applicant provides a filler to be incorporated into a resin composition used for dental applications.

Citation List

[Patent Literature]

  Patent Literature 1: JP2021-155399 (A)
  Patent Literature 2: JP2021-172577 (A)

Summary

Technical Problem

Resin compositions used for dental applications are required to exhibit aesthetic properties. For example, dental resin compositions are required not only to have high strength but also to exhibit excellent appearance characteristics such as gloss obtained by polishing (hereinafter, referred to as "polishability"). The inventors of the present application have considered that a filler disclosed in Patent Literature 2 does not sufficiently achieve both polishability and fillability. As a result of extensive and intensive studies, the inventors have found that, in order to achieve both strength and polishability, the incorporation of a large amount of filler is enabled by limiting the upper limit of the specific surface area and by reducing the linseed oil absorption amount per unit surface area, so that polishability is improved.

In addition, when a material is used as a dental material, the material is required to exhibit X-ray radiopacity as a desirable physical property. Here, (meth)acrylic acid-based resin materials generally used for dental materials have a refractive index of about 1.50. Metal oxides (such as zirconia) having X-ray radiopacity have a high refractive index. Even when metal oxides are simply added as fillers, adjustment of the refractive index to about 1.50 is not easy. When such metal oxides are added while maintaining the refractive index, sufficient X-ray radiopacity is not exhibited. Furthermore, exposure of metal oxides having X-ray radiopacity at the surface raises concerns such as deterioration in polishability, reduced reactivity with silane coupling agents, and deterioration in stability.

The present disclosure has been completed in view of the above circumstances. An object of the present disclosure is to provide a particulate material used as a filler for a dental material, and a dental material using the particulate material.

Solution to Problem

A particulate material of the present disclosure that solves the above-described problem is formed of an aggregate that is composed of primary particles having a volume-average particle diameter of 1 nm to 20 nm and including two or more metal elements, the aggregate having a surface composed of silica, wherein

  some of the primary particles each contain a heavy element having an atomic number of 38 or greater in the form of a metal oxide;
  at least some of the remaining primary particles are particles composed of silica; and
  the particulate material has:
  a specific surface area of 180 $m^2$/g or less;
  a linseed oil absorption amount per unit volume (100 $cm^3$) of 200 g or less;
  a linseed oil absorption amount per unit surface area ($m^2$) of 0.02 g or less;

a light transmittance of more than 20%; and
a mass of the heavy element of 4% to 60% based on a total mass.

The use of the aggregate makes it possible to enhance strength while achieving optical performance comparable to that of materials having a small particle diameter. The surface of the aggregate is composed of silica, thereby allowing optional functional groups to be introduced onto the surfaces by using a silane coupling agent or the like, and suppressing surface exposure of heavy elements and the like.

Advantageous Effects

The particulate material of the present disclosure having the above configuration achieves both polishability and fillability when used in a dental material.

Brief Description of Drawing

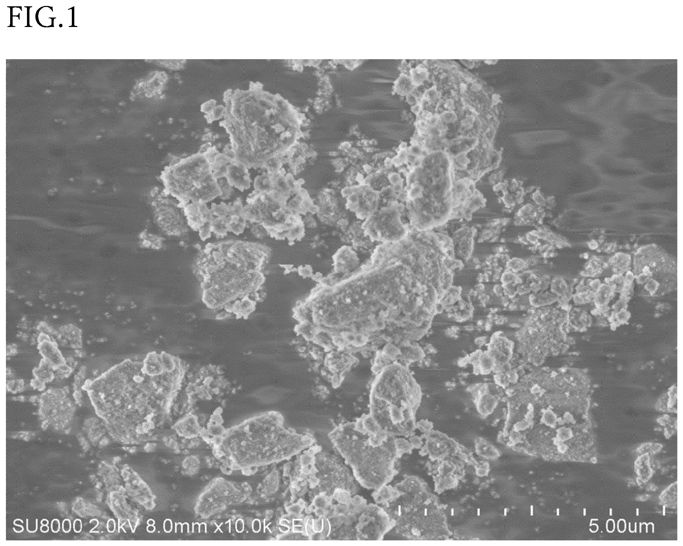

Fig. 1 is an SEM photograph of a filler 1 in Example.

Description of Embodiments

A particulate material of the present disclosure and a dental material using the particulate material will be described in detail below on the basis of embodiments. The particulate material of the present embodiment provides a resin composition having high light transmittance when dispersed in a transparent resin material. The resin composition having high light transmittance is suitably applicable to dental materials such as denture materials and dental filling materials.

(Particulate material)

The particulate material of the present embodiment is formed of an aggregate that is composed of primary particles having a volume-average particle diameter of 1 nm to 20 nm and including two or more metal elements, the aggregate having a surface composed of silica. Some of the primary particles (hereinafter, referred to as "first primary particles") each contain a heavy element having an atomic number of 38 or greater in the form of a metal oxide. At least some of the remaining primary particles (hereinafter, referred to as "second primary particles") are composed of silica. That is, the primary particles constituting the aggregates include the first primary particles, the second primary particles, and other primary particles that may optionally be included as necessary (hereinafter, referred to as "other primary particles").

The particulate material is preferably formed of an aggregate in which a plurality of types of primary particles described above are aggregated by being bonded to each other by dehydration condensation or by being fused to each other. OH groups are present on the surfaces of the metal oxides contained in the primary particles, and dehydration condensation proceeds between adjacent primary particles, or the metal oxides melt and become fused to each other.

The particle diameter of each aggregate is not particularly limited. Since the primary particles are bonded or fused to each other, the primary particles are firmly bonded to each other, thereby improving the mechanical strength of the particulate material. In addition, the shape of the aggregate is not particularly limited. However, a crushed shape is preferable. The crushed shape refers to a shape having a surface formed by crushing, and for example, as shown in Fig. 1, refers to a shape having an angular surface when observed by SEM. When the crushed shape is adopted, physical properties such as flexural strength are improved when the aggregate is used in a resin composition.

The volume-average particle diameter of the aggregates is preferably 0.1 $\mu$m or more and 10 $\mu$m or less. Examples of the lower limit value include 0.2 $\mu$m, 0.3 $\mu$m, 0.5 $\mu$m, and 1.0 $\mu$m, and examples of the upper limit value include 8.0 $\mu$m, 5.0 $\mu$m, and 3.0 $\mu$m. The lower limit values and the upper limit values may be combined in any combination. In a case where the particle diameter of the aggregates falls within a range equal to or more than the above-described lower limit value, the strength is improved when the aggregate is applied to a resin composition. In addition, in a case where the particle diameter of the aggregates falls within a range equal to or less than the above-described upper limit value, the particulate material is appropriately dispersed in a resin material or the like. When aggregates having large particle diameters and aggregates having small particle diameters are mixed as separate aggregates, a packing fraction of the aggregates incorporated into the resin composition is improved.

The specific surface area of the aggregate is 180 m$^2$/g or less. In particular, examples of the upper limit value include 160 m$^2$/g, 150 m$^2$/g, 130 m$^2$/g, and 100 m$^2$/g, and examples of the lower limit value include 10 m$^2$/g, 15 m$^2$/g, 20 m$^2$/g, and 30 m$^2$/g. The lower limit values and the upper limit values may be combined in any combination. The specific surface area is a value measured by a BET method using nitrogen gas.

The volume-average particle diameter of the primary particles constituting the aggregates is exemplified by lower limit values of 3 nm, 5 nm, 9 nm, 11 nm, and 14 nm and by upper limit values of 18 nm and 16 nm. The lower limit values and the upper limit values may be combined in any combination. Furthermore, the volume-average particle diameter ranges are also independently set for each of the first primary particles, the second primary particles, and the other primary particles. The volume-average particle diameter of the primary particles in the aggregated particles is measured by a method in which the aggregated particles are embedded in a resin, a cross section of the aggregated particles is exposed by polishing and ion milling, the exposed cross section is observed by SEM or TEM, and image analysis is performed.

The metal elements included in the primary particles are not particularly limited. However, metal elements that form oxides having high stability are preferable. In particular, examples of the metal elements include aluminum, zirconium, titanium, and silicon, which are capable of forming oxides having high stability against moisture. Two or more metal elements are present within the aggregate as individual metal oxides, or as composite oxides of two or more metal elements.

The two metal elements may be aggregated as primary particles having a plurality of compositions, in which each of the primary particles constituting an aggregate contains a single compound, and alternatively, a single primary particle may contain a plurality of metal elements or a plurality of compounds.

Each first primary particle contains a heavy element. The heavy element refers to an element having an atomic number of 38 or greater, and, while not particularly limited, is preferably selected from zirconium, barium, and titanium. The first primary particle may contain, in the same particle, the heavy element together with other metal elements as described above, for example, in the form of composite oxides. The content of the heavy element is 4% or more and 60% or less based on the total mass of the particulate material, with the lower limit value selectable from 5%, 7%, 10%, and 14% and the upper limit value selectable from 55%, 50%, 40%, and 30%. The lower limit values and the upper limit values may be combined in any combination.

The inclusion of second primary particles composed of silica improves polishability when the second primary particles are applied to a resin composition. Here, being composed of silica means that the second primary particles have a silica content of 80% or more on a mass basis, preferably 90% or more, more preferably 95% or more, further preferably 99%, and particularly preferably 100% (excluding unavoidable impurities).

The second primary particles have an upper limit value of about 95%, 90%, or 85%, and a lower limit value of about 50%, 30%, 20%, or 10%, based on the total mass. The upper limit values and the lower limit values may be combined in any combination.

Furthermore, primary particles composed of a compound consisting of elements other than the metal elements to be included may also be contained. The elements other than the metal elements may be contained in an amount not exceeding an amount at which the metal elements constitute a main component. Here, the amount at which the metal elements constitute a main component means that the metal elements are contained in an amount of 50 mass% or more, preferably 70 mass% or more, and more preferably 90 mass% or more.

Particles that neither contain a heavy element nor are composed of silica may also be included as other primary particles. Examples of such other primary particles include those containing metal elements as described above.

The metal elements may be present not only in the form of metal oxides, but also in the form of nitrides, carbides, or the like. Specific examples of the internal composition include aluminum oxide, zirconia, titania, silica, and composite oxides thereof. As the aluminum oxide, γ-alumina or boehmite may be used.

The surface of the aggregate is composed of silica. That is, a configuration in which the first primary particles (and other primary particles, if included) form an interior portion (referred to as "core" as appropriate), and the surface of the core is coated with silica, is adopted. The silica coating may be performed after formation of the aggregate, or may be performed while the particles are in the form of primary particles.

Here, the surface of the aggregate being composed of silica means that the area of a portion of the surface of the aggregate covered with silica (hereinafter, sometimes referred to as "silica portion" or "coating layer") is at least 50%, preferably 70%, 75%, 80%, 85%, 90%, 95%, or 99%, and more preferably, the surface is entirely covered with silica without leaving exposed portions (100%).

In order to cover the surface with silica without leaving exposed portions, coating with a layer formed through a chemical reaction is effective, and, for example, a silica coating is formed by hydrolyzing, and dehydrating and condensing tetraethoxysilane. On the other hand, when a silica coating layer containing an amount of silica required to produce a filler having a refractive index suitable for dental materials is formed solely by the above-described chemically bonded layer, the bonding between the silica coating layers becomes strong, so that the polishability deteriorates, and the transparency also deteriorates. The area of the portion covered with silica is calculated by directly measuring silica through direct observation using an electron microscope, or is indirectly calculated by measuring the amount of metal elements other than silica.

The particulate material has a light transmittance of 20% or more, preferably 25% or more, 30% or more, 35% or more, 40% or more, or 50% or more.

The light transmittance is measured as follows. First, test samples are placed into test liquids whose refractive indices were adjusted at intervals of 0.005, and OD values are measured in a total of five test liquids, including a test liquid exhibiting the highest transparency by visual observation (the refractive index of this test liquid is defined as the refractive index of the test sample) and test liquids having refractive indices of ±0.005 and ±0.01 relative thereto. The refractive indices of the obtained test liquids and OD values in the test liquids are plotted, and the light transmittance is calculated from the OD value at the vertex of a quadratic curve fitted to the plot. The OD value is defined as the value measured using light having a wavelength of 589 nm for a sample suspension (prepared by suspending 0.1 g of the test sample in 2 mL of the test liquid) in a cell having a diameter of 15 mm.

The silica covering the surface, when converted to $SiO_2$, has a lower limit value selectable from 40%, 50%, or 60% and an upper limit value selectable from 95%, 90%, and 85%, based on the total mass of the particulate material. The lower limit values and the upper limit values may be combined in any combination.

The silica portion formed on the surface is a layer present on the surface of the aggregate. The thickness of the coating layer is not particularly limited. However, the surface of the aggregate is preferably covered substantially without gaps. When a coating layer is provided, the surface may be coated in a state in which primary particles are aggregated, such that the primary particles are directly aggregated with each other (i.e., the coating layer is formed after the primary particles have been aggregated), or alternatively, the coating layer may be interposed between aggregated primary particles (i.e., the coating layer is formed on the surfaces of the primary particles in the primary-particle state, and then the primary particles are aggregated to form an aggregate). Desirably, the coating layer is bonded to the surfaces of the primary particles either by covalent bonding or by physical bonding such as intermolecular force bonding. Provision of a silica coating layer makes it possible to suppress exposure of the highly active surface of zirconia, thereby suppressing deterioration of a resin when the material is dispersed and used in the resin, and also suppressing temporal changes of organic components for surface modification. The organic material constituting the coating layer is preferably a condensate of a silane compound. When a silane compound having two or more SiOR groups is used, a coating layer composed of the condensate is formed. The condensate of the silane compound is produced by allowing the silane compound to undergo a condensation reaction while the silane compound is in contact with the surfaces of the primary particles (before or after the primary particles are aggregated). The primary particles are composed of an inorganic material and typically have OH groups on the surfaces of the primary particles. Thus, the above silane compound reacts with OH groups present on the surfaces of the primary particles to form covalent bonds.

Functional groups may be introduced onto the surface of the silica portion. The functional groups to be introduced are not particularly limited. However, when the material is to be dispersed in a resin material, functional groups having high affinity for the resin material are preferably introduced. For example, when acrylic acid and esters thereof, methacrylic acid and esters thereof, epoxy resins, or the like are used as a resin material, introduction of acrylic groups, methacrylic groups, epoxy groups, or the like makes it possible to react with the resin material and form a strong bond.

In addition, a functional group represented by formula (1) : $-OSiX^1X^2X^3$ and a functional group represented by formula (2): $-OSiY^1Y^2Y^3$ may be introduced onto the surface of the silica portion. Here, in formulas (1) and (2), $X^1$ is a phenyl group, a vinyl group, an epoxy group, a methacrylic group, an amino group, a ureido group, a mercapto group, an isocyanate group, or an acrylic group; $X^2$ and $X^3$ are each independently selected from $-OSiR_3$ and $-OSiY^4Y^5Y^6$; $Y^1$ is R; and $Y^2$ and $Y^3$ are each independently selected from R and $-OSiY^4Y^5Y^6$. $Y^4$ is R; $Y^5$ and $Y^6$ are each independently selected from R and $-OSiR_3$; and R is independently selected from alkyl groups having 1 to 3 carbon atoms. Any one of $X^2$, $X^3$, $Y^2$, $Y^3$, $Y^5$, and $Y^6$ may be bonded to any one of $X^2$, $X^3$, $Y^2$, $Y^3$, $Y^5$, and $Y^6$ of an adjacent functional group via -O-.

The refractive index of the particulate material is controlled by adjusting the ratio between the amount of the silica portion and the amount of other metal oxides, and by introducing functional groups into the silica portion. Since the coated particles have a small particle diameter, the refractive index is adjusted while maintaining transparency. The refractive index of the particulate material preferably differs from that of the transparent resin material used in a dental material described below by -0.1 to 0.1.

The particulate material has a linseed oil absorption amount of 200 g or less per unit volume ($100 cm^3$), and specific examples of the upper limit include 170 g, 160 g, and 150 g. This value is reduced by improving the packing fraction of the particulate material or improving the sphericity of the particles. The unit volume is a value calculated as (apparent volume) × (1 - porosity).

The linseed oil absorption amount per unit surface area ($m^2$), calculated from the surface area of the particulate material (a value obtained through measurement by a BET method using nitrogen gas), is 0.02 g or less, and specific examples of the upper limit include 0.018 g, 0.016 g, and 0.014 g. This value is reduced by improving the packing fraction of the particulate material or improving the sphericity of the particles.

The particulate material may contain an inorganic particulate material that has a volume-average particle diameter of 1 nm to 200 nm and is dispersed into primary particles. The inorganic particulate material may be contained in an amount of more than 0% and up to about 90%, based on the total mass of the particulate material. Examples of the lower limit value of the content of the inorganic particulate material include 2%, 5%, and 10%, and examples of the upper limit value include 60%, 50%, and 40%. The lower limit values and the upper limit values may be combined in any combination. When the inorganic particulate material is contained within the above-described range, the viscosity of the resin composition is reduced, and the toughness of the cured resin product containing the inorganic particulate material is improved.

The inorganic particulate material preferably has the functional group represented by formula (1): -OSiX$^1$X$^2$X$^3$ and the functional group represented by formula (2): -OSiY$^1$Y$^2$Y$^3$ bonded to the surfaces of silica particles. Since formulas (1) and (2) are the same as those described above, the description is omitted.

(Method for producing particulate material)

A method for producing a particulate material according to the present embodiment is a production method capable of suitably producing the above-described particulate material. The method for producing the particulate material of the present embodiment includes a dispersion step, a coating step, and other steps that are optionally selected as necessary. Examples of such other steps include an aggregation step, a modification step, and a particle size distribution adjustment step.

The dispersion step is a step of dispersing particles having an internal composition (core particles, which form particles corresponding to first primary particles and, if present, other primary particles by undergoing the coating step described below) in a liquid dispersion medium to prepare a liquid dispersion. The core particles are obtained by conventional methods. For example, the core particles are produced by reacting compounds that serve as precursors of the internal composition. For example, when zirconia is used as the internal composition, a zirconia sol is used. When boehmite is used, aluminum hydroxide adjusted to have an appropriate particle diameter by pulverization or the like is used as a precursor, and core particles composed of boehmite are obtained by subjecting the precursor to hydrothermal treatment.

In addition, aluminum oxide adjusted to have an appropriate particle diameter is used as a precursor, and core particles are obtained by heating the precursor in an acidic or alkaline aqueous solution. Colloidal silica is also regarded as coated particles in which core particles composed entirely of silica are coated with a surface composition composed of silica, and therefore, the addition of colloidal silica is also included in the dispersion step. As the core particles, a mixture of a plurality of types of materials is used, and alternatively, core particles composed of a plurality of types of materials are added sequentially.

The coating step is a step of adding a precursor, which is a compound that forms a surface composition (silica) through a reaction, to the obtained liquid dispersion to generate the surface composition, thereby coating and forming the surfaces of core particles with the surface composition to obtain coated particles. In cases where silica particles such as colloidal silica (corresponding to second primary particles) are added in the dispersion step, the colloidal silica is equivalent to coated particles in which the surfaces of core particles composed of silica are coated with a surface composed of silica, and thus functions as coated particles even in the absence of the coating step.

The coating step may be performed at one time or may be performed a plurality of times. Further, when the dispersion step is performed a plurality of times, the coating step may be performed in the course of the dispersion step. The ratio between the internal composition and the surface composition is controlled by adjusting the amount of the core particles and the amount of the precursor to be added.

As the precursor, any compound may be used. For example, tetraethoxysilane is used as the precursor. Tetraethoxysilane readily forms silica in the presence of water. A so-called sol-gel method in which tetraethoxysilane is hydrolyzed under an acidic or basic atmosphere is employed.

The aggregation step is a step that is performed after the coating step, and is a step of heating the coated particles obtained in the coating step to cause aggregation thereof. In this step, aggregates are formed. The coated particles correspond to primary particles of the aggregates. The obtained aggregates are subjected to a pulverization operation and a classification operation so as to achieve a required particle size distribution. The heating temperature in the aggregation step is a temperature at which dehydration condensation or softening occurs between the coated particles. For example, temperatures exceeding 250°C, temperatures of 450°C or higher, and temperatures of 500°C or higher are exemplified. Heating in this temperature range improves the strength of a particulate material to be obtained.

The modification step is a step that is performed after the coating step, and is a step of modifying the coated particles obtained in the coating step by bringing a silane compound into contact with the surfaces of the coated particles. When combined with the aggregation step, the modification step may be performed either before or after the aggregation step.

(Dental material)

The dental material of the present embodiment includes the above-described particulate material and a transparent resin material in which the particulate material is dispersed. The content of the particulate material is 10% or more and 80% or less, based on the total volume thereof.

Since the applicable particulate material is described above, further description thereof is omitted. As the transparent resin material, conventional resin materials such as thermoplastic resins and thermosetting resins may be selected. Examples thereof include acrylic acid or acrylic acid ester and derivatives thereof, epoxy resin, polyimide, polycarbonate, polyethylene terephthalate, polybutylene terephthalate, polymethyl methacrylate, vinyl chloride, polypropylene, and polyethylene. The transparent resin material represents a concept that includes, in addition to polymeric materials, precursors such as monomers prior to polymerization.

A method for dispersing the particulate material in the transparent resin material is not particularly limited. For example, when a thermoplastic resin is used as the transparent resin material, a transparent resin composition is obtained by mixing and kneading the particulate material with the transparent resin material in a heated and molten state, or by mixing the particulate material with a precursor of the transparent resin material (transparent resin precursor material: a monomer, a prepolymer, or the like) and subsequently performing a polymerization reaction. When the transparent resin material is a thermosetting resin, a transparent resin precursor is mixed with the particulate material and subsequently the mixture is cured. A mixture of the transparent resin precursor prior to polymerization and the particulate material is also included in the dental material of the present embodiment. When a photopolymerizable material is used as the transparent resin precursor material, a photopolymerizable transparent resin composition is provided. The resin composition after polymerization is expected to exhibit high transparency, a low coefficient of thermal expansion (CTE), and a high elastic modulus.

Examples

The particulate material of the present disclosure is described in detail below on the basis of the following Examples. First, the production of fillers used for the tests is described. Fillers 1 to 8 correspond to the particulate materials of the present disclosure (Examples), whereas fillers 9 to 17 correspond to particulate materials falling outside the scope of the present disclosure (Comparative examples).

(Preparation of sample)

·Filler 1

300 g of isopropyl alcohol (IPA) and 50 g of tetraethoxysilane (Ethyl Silicate 28, COLCOAT CO.,LTD.) were mixed, and then, 500 g of zirconium sol (particle diameter: 14 nm, solid content: 20%, DAIICHI KIGENSO KAGAKU KOGYO CO., LTD.) was added and stirred. The mixture was allowed to stand at 44°C for 72 hours (dispersion step, coating step).

Then, 2150 g of SNOWTEX OS (acidic colloidal silica sol, particle diameter: 9 nm, solid content: 20 mass%, Nissan Chemical Corporation) was added and stirred, and the mixture was allowed to stand at 44°C for 24 hours. A 5% aqueous ammonia solution was added to adjust the pH to 8-9. The obtained liquid was a flowable sol (dispersion step). The obtained sol was dried at 140°C for 14 hours using a hot-air dryer (aggregation step).

The dried material was adjusted so as to have a D50 of 2 $\mu$m using a mixer and a dry jet mill (particle size distribution adjustment step), thereby obtaining a material (having a crushed shape, Fig. 1). The material was calcined at 1010°C for 6 hours using an electric furnace (calcination step). The BET specific surface area of the powder obtained after calcination was 102 m$^2$/g.

10 parts by mass of 3-methacryloxypropyltrimethoxysilane (KBM503 manufactured by Shin-Etsu Chemical Co., Ltd.) were added to the powder obtained after calcination, and the mixture was stirred using a mixer to achieve uniform adhesion to the surface of the calcined powder. In the filler 1 described in this example, 3-methacryloxypropyltrimethoxysilane was reacted in an amount of 4 $\mu$mol per m$^2$ of surface area. Thereafter, the mixture was allowed to stand at 44°C for 24 hours, and then dried at 105°C for 6 hours using a hot-air dryer to perform surface treatment, thereby obtaining the filler 1, which was a test sample of the present test (modification step). The obtained filler 1 was produced by introducing 3-methacryloxypropyl groups onto the surfaces of aggregates obtained by aggregating and sintering coated particles in which core particles composed of zirconia were coated with silica, and silica particles (corresponding to coated particles in which silica core particles were coated with silica). The introduction of 3-methacryloxypropyl groups was performed in order to enhance affinity for a formulated resin described below. Depending on the type of resin to be mixed, the type of functional group preferably introduced differs, and is appropriately selected.

·Filler 2

The filler 2 as the test sample in the present test was obtained by the same process as that used for the filler 1, except that the material was calcined at 1020°C for 6 hours using an electric furnace (the BET specific surface area of the powder obtained after calcination was 63 m$^2$/g) in the calcination step, and 6 parts by mass of 3-methacryloxypropyltrimethoxysilane were added to the calcined powder in the modification step.

·Filler 3

The filler 3 as the test sample in the present test was obtained by the same process as that used for the filler 1, except that the material was calcined at 1040°C for 6 hours using an electric furnace (the BET specific surface area of the powder obtained after calcination was 25 m$^2$/g) in the calcination step, and 3 parts by mass of 3-methacryloxypropyltrimethoxysilane were added to the calcined powder in the modification step.

·Filler 4

The filler 4 as the test sample in the present test was obtained by the same process as that used for the filler 1, except that the material was calcined at 850°C for 6 hours using an electric furnace (the BET specific surface area of the powder obtained after calcination was 159 m$^2$/g) in the calcination step, and 16 parts by mass of 3-methacryloxypropyltrimethoxysilane were added to the calcined powder in the modification step.

·Filler 5

The filler 5 as the test sample in the present test was obtained by the same process as that used for the filler 1, except that the amount of SNOWTEX OS was set to 1525 g in the dispersion step, the material was calcined at 850°C for 6 hours using an electric furnace (the BET specific surface area of the powder obtained after calcination was 155 m$^2$/g) in the calcination step, and 15 parts by mass of 3-methacryloxypropyltrimethoxysilane were added to the calcined powder in the modification step.

·Filler 6

The filler 6 as the test sample in the present test was obtained by the same process as that used for the filler 1, except that the amount of SNOWTEX OS was set to 2750 g in the dispersion step, the material was calcined at 1010°C for 6 hours using an electric furnace (the BET specific surface area of the powder obtained after calcination was 47 m$^2$/g) in the calcination step, and 5 parts by mass of 3-methacryloxypropyltrimethoxysilane were added to the calcined powder in the modification step.

·Filler 7

The filler 7 as the test sample in the present test was obtained by the same process as that used for the filler 1, except that the amount of SNOWTEX OS was set to 3660 g in the dispersion step, the material was calcined at 1010°C for 6 hours using an electric furnace (the BET specific surface area of the powder obtained after calcination was 43 m$^2$/g) in the calcination step, and 4 parts by mass of 3-methacryloxypropyltrimethoxysilane were added to the calcined powder in the modification step.

·Filler 8

The filler 8 as the test sample in the present test was obtained by the same process as that used for the filler 1, except that the amount of SNOWTEX OS was set to 415 g in the dispersion step, the material was calcined at 850°C for 6 hours using an electric furnace (the BET specific surface area of the powder obtained after calcination was 150 m$^2$/g) in the calcination step, and 15 parts by mass of 3-methacryloxypropyltrimethoxysilane were added to the calcined powder in the modification step.

·Filler 9

The filler 9 as the test sample in the present test was obtained by the same process as that used for the filler 1, except that the material was calcined at 1080°C for 6 hours using an electric furnace (the BET specific surface area of the powder obtained after calcination was 12 m$^2$/g) in the calcination step, and 1 part by mass of 3-methacryloxypropyltrimethoxysilane was added to the calcined powder in the modification step.

·Filler 10

The filler 10 as the test sample in the present test was obtained by the same process as that used for the filler 1, except that the amount of tetraethoxysilane was set to 105 g, and 500 g of Alumisol-10A (particle diameter: 10 nm, solid content: 10 mass%, manufactured by Kawaken Fine Chemicals Co., Ltd., crystal form: pseudoboehmite) was used in place of the zirconia sol in the dispersion step and the coating step, the amount of SNOWTEX OS was set to 500 g in the dispersion step, the material was calcined at 1090°C for 6 hours using an electric furnace (the BET specific surface area of the powder obtained after calcination was 100 m$^2$/g) in the calcination step, and 10 parts by mass of 3-methacryloxypropyltrimethoxysilane were added to the calcined powder in the modification step.

·Filler 11

The filler 11 as the test sample in the present test was obtained by the same process as that used for the filler 10, except that the material was calcined at 1100°C for 6 hours using an electric furnace (the BET specific surface area of the powder obtained after calcination was 50 m$^2$/g) in the calcination step, and 5 parts by mass of 3-methacryloxypropyltrimethoxysilane were added to the calcined powder in the modification step.

·Filler 12

0.4 parts by mass of 3-methacryloxypropyltrimethoxysilane were added to 100 parts by mass of a particulate material composed of silica (volume-average particle diameter: 2 $\mu$m, SO-C6 manufactured by ADMATECHS COMPANY LIMITED), and the mixture was stirred using a mixer. Thereafter, the mixture was allowed to stand at 25°C for 72 hours, followed by drying at 105°C for 6 hours using a hot-air dryer to perform surface treatment, thereby obtaining the filler 12.

·Filler 13

20 parts by mass of 3-methacryloxypropyltrimethoxysilane were added to 100 parts by mass of hydrophilic fumed silica (AEROSIL200 manufactured by NIPPON AEROSIL CO., LTD.), and the mixture was stirred using a mixer. Thereafter, the mixture was allowed to stand at 25°C for 72 hours, followed by drying at 105°C for 6 hours using a hot-air dryer to perform surface treatment, thereby obtaining the filler 13.

·Filler 14

The filler 14 as the test sample in the present test was obtained by the same process as that used for the filler 1, except that a zirconia sol having a particle diameter of 25 nm (particle diameter: 25 nm, solid content: 20%, manufactured by DAIICHI KIGENSO KAGAKU KOGYO CO., LTD.) was used in place of the zirconia sol having a particle diameter of 14 nm in the dispersion step and the coating step, the material was calcined at 850°C for 6 hours using an electric furnace (the BET specific surface area of the powder obtained after calcination was 243 m$^2$/g) in the calcination step, and 24 parts by mass of 3-methacryloxypropyltrimethoxysilane were added to the calcined powder in the modification step.

·Filler 15

The filler 15 as the test sample in the present test was obtained by the same process as that used for the filler 1, except that a zirconia sol having a particle diameter of 25 nm (particle diameter: 25 nm, solid content: 20%, manufactured by DAIICHI KIGENSO KAGAKU KOGYO CO., LTD.) was used in place of the zirconia sol having a particle diameter of 14 nm in the dispersion step and the coating step, the material was calcined at 1020°C for 6 hours using an electric furnace (the BET specific surface area of the powder obtained after calcination was 68 m$^2$/g) in the calcination step, and 7 parts by mass of 3-methacryloxypropyltrimethoxysilane were added to the calcined powder in the modification step.

·Filler 16

The filler 16 as the test sample in the present test was obtained by the same process as that used for the filler 1, except that the amount of isopropyl alcohol was set to 1000 g, the amount of tetraethoxysilane was set to 1500 g, SNOWTEX OS was not added in the dispersion step and the coating step, the material was calcined at 850°C for 6 hours using an electric furnace (the BET specific surface area of the powder obtained after calcination was 2 m$^2$/g) in the calcination step, and 0.2 parts by mass of 3-methacryloxypropyltrimethoxysilane were added to the calcined powder in the modification step. As a result, a particulate material that did not include primary particles composed of silica (second primary particles) was obtained.

·Filler 17

After 100 g of ion exchanged water and 100 g of SNOWTEX OS were mixed, 75 g of zirconyl nitrate solution (solid content: 20%, manufactured by TAIYO KOKO CO., LTD.) was added and stirred. Thereafter, 152 g of a 1% aqueous ammonia solution was added, and the mixture was allowed to stand at room temperature for 72 hours (dispersion step, coating step).

Subsequently, 230 g of SNOWTEX OS was added and stirred, and the mixture was allowed to stand at 44°C for 24 hours. A 5% aqueous ammonia solution was added to adjust the pH to 8-9. The obtained liquid was a flowable sol (dispersion step). The obtained sol was dried at 140°C for 14 hours using a hot-air dryer (aggregation step). Thereafter, except that 19 parts by mass of 3-methacryloxypropyltrimethoxysilane were added to the calcined powder in the modification step, the filler 17 as the test sample in the present test was obtained by the same process as that used for the filler 1. As a result, a particulate material that did not include zirconia (corresponding to a heavy element) as primary particles (first primary particles) was obtained.

(Linseed oil absorption amount and specific surface area)

Each test sample in an amount of 5 to 10 g was weighed out and measured. Linseed oil was added little by little, while an operation using an Awatori Rentaro was performed at 2000 rpm at atmospheric pressure for 1 minute, followed by an operation at 2000 rpm at 7.5 kPa for 1 minute. The above operations were repeated, and the amount of linseed oil required just before the mixture became paste-like was used to calculate the linseed oil absorption amount according to the following equations. A value calculated according to the composition ratio was used, with specific gravities of silica, zirconia, and alumina taken as 2.38 $g/cm^3$, 6.00 $g/cm^3$, and 3.36 $g/cm^3$, respectively. The specific surface area was measured by the BET method using nitrogen gas.

(Linseed oil absorption amount [g/100 g]) = (total mass of added linseed oil) / (mass of used filler) $\times$ 100

(Linseed oil absorption amount [g/100 $cm^3$]) = (linseed oil absorption amount [g/100 g]) $\times$ specific gravity [g/$cm^3$]

(Linseed oil absorption amount [g/$m^2$]) = (linseed oil absorption amount [g/100 g]) / (specific surface area [$m^2$/g] $\times$ 100)

(Polishability)

10 g of each test sample and 10 g of a formulated resin were mixed by an operation using an Awatori Rentaro at 2000 rpm at atmospheric pressure for 1 minute, followed by an operation at 2000 rpm under 3 kPa for 3 minutes. The obtained slurry-like resin composition was poured into a mold having dimensions of 2.6 cm $\times$ 2.6 cm and a height of 6 mm, and cured in a UV oven. For the fillers 10 and 13, a slurry-like resin composition could not be prepared.

The obtained cured product was polished for 2 minutes using a pressure-type polishing machine (Doctor-lap manufactured by MARUTO INSTRUMENT CO.,LTD.) under polishing conditions of LOAD ADJUST 0.35, BALANCER 0.35, HEAD SPEED 3.2, and SPEED 20, using #400 abrasive paper.

Then, polishing was performed under the same polishing conditions, except that the abrasive paper was replaced with #2000 abrasive paper. The sample was checked every 10 seconds to measure the time required until gloss was observed over the entire polished surface.

The used formulated resin was a mixture of 60 parts by mass of urethane dimethacrylate (SigmaAldrich), 40 parts by mass of triethylene glycol dimethacrylate (SHIN-NAKAMURA CHEMICAL Co., Ltd.), 0.3 parts by mass of camphorquinone (SigmaAldrich), and 1 part by mass of ethyl 4-dimethylaminobenzoate (Tokyo Chemical Industry Co., Ltd.).

(Refractive index and light transmittance)

The refractive index and the light transmittance are measured by the method described in the embodiments. Hereinafter, the processes are specifically described using a filler adjusted so as to have a refractive index of 1.51 as an example.

The filler adjusted so as to have a refractive index of 1.51 exhibits the highest transparency when immersed in a test liquid having a refractive index of 1.51. Therefore, test liquids having refractive indices of 1.500, 1.505, 1.510, 1.515, and 1.520 were prepared using cyclohexane, toluene, and bromonaphthalene, as test liquids having refractive indices differing by $\pm$0.005 and $\pm$0.010 from 1.51.

0.1 g of a test sample was added to 2 mL of each test liquid, and the mixture was placed in a cell having a diameter of 15 mm. The optical density (OD value) in the up-down direction was measured at a wavelength of 589 nm. The OD values in the test liquids were plotted with respect to the refractive indices and fitted to a quadratic curve, and the light transmittance was calculated from the OD value at the vertex (the point having the lowest OD value).

**EP 4 785 923 A1**

(Results)

Table 1 shows the refractive index, specific surface area, linseed oil absorption amount, content ratio of zirconium (elements having an atomic number of 38 or greater), zirconia content (expressed in terms of elemental Zr), light transmittance, and polishability.

[Table 1]

| | | Example | Example | Example | Example |
|---|---|---|---|---|---|
| | | Filler 1 | Filler 2 | Filler 3 | Filler 4 |
| Core particle | | Zirconia | Zirconia | Zirconia | Zirconia |
| Refractive index | | 1.51 | 1.51 | 1.51 | 1.51 |
| Zirconia primary particle diameter [nm] | 1 to 20 | 14 | 14 | 14 | 14 |
| Specific surface area [$m^2$/g] | 180 or less | 102 | 63 | 25 | 159 |
| Linseed oil absorption amount [g/100 g] | 200 or less | 45 | 38 | 31 | 60 |
| Linseed oil absorption amount [g/100 $cm^3$] | | 121 | 100 | 83 | 160 |
| Linseed oil absorption amount per surface area [$g/m^2$] | 0.02 or less | 0.0044 | 0.0060 | 0.0124 | 0.0038 |
| Zirconia content | 4 to 60 | 18% | 18% | 18% | 18% |
| Transparency [%] | Higher than 20 | 74% | 37% | 25% | 91% |
| Polishability [s] (time until gloss develops) | | 20 | 30 | 60 | 20 |

[Table 1] (Continued)

| | | Example | Example | Example | Example |
|---|---|---|---|---|---|
| | | Filler 5 | Filler 6 | Filler 7 | Filler 8 |
| Core particle | | Zirconia | Zirconia | Zirconia | Zirconia |
| Refractive index | | 1.53 | 1.50 | 1.49 | 1.65 |
| Zirconia primary particle diameter [nm] | 1 to 20 | 14 | 14 | 14 | 14 |
| Specific surface area [$m^2$/g] | 180 or less | 155 | 47 | 43 | 150 |
| Linseed oil absorption amount [g/100 g] | 200 or less | 58 | 38 | 37 | 48 |
| Linseed oil absorption amount [g/100 $cm^3$] | | 161 | 100 | 95 | 164 |
| Linseed oil absorption amount per surface area [$g/m^2$] | 0.02 or less | 0.0037 | 0.0081 | 0.0086 | 0.0032 |
| Zirconia content | 4 to 60 | 24% | 15% | 12% | 50% |
| Transparency [%] | Higher than 20 | 91% | 45% | 49% | 90% |
| Polishability [s] (time until gloss develops) | | 20 | 40 | 40 | 20 |

[Table 1] (Continued)

| | | Comp. Ex. | Comp. Ex. | Comp. Ex. | Comp. Ex. |
|---|---|---|---|---|---|
| | | Filler 9 | Filler 10 | Filler 11 | Filler 12 |
| Core particle | | Zirconia | Alumina | Alumina | Spherical silica (particle diameter: 2 $\mu$m) |
| Refractive index | | 1.51 | 1.51 | 1.51 | 1.46 |

(continued)

| | | Comp. Ex. | Comp. Ex. | Comp. Ex. | Comp. Ex. |
|---|---|---|---|---|---|
| | | Filler 9 | Filler 10 | Filler 11 | Filler 12 |
| Zirconia primary particle diameter [nm] | 1 to 20 | 14 | - | - | - |
| Specific surface area [m$^2$/g] | 180 or less | 12 | 100 | 50 | 1.77 |
| Linseed oil absorption amount [g/100 g] | 200 or less | 30 | 102 | 67 | 15 |
| Linseed oil absorption amount [g/100 cm$^3$] | | 80 | 264 | 174 | 36 |
| Linseed oil absorption amount per surface area [g/m$^2$] | 0.02 or less | 0.0250 | 0.0102 | 0.0134 | 0.0847 |
| Zirconia content | 4 to 60 | 18% | 0% | 0% | 0% |
| Transparency [%] | Higher than 20 | 10% | 94% | 38% | 98% |
| Polishability [s] (time until gloss develops) | | 120 | - | 30 | No gloss was observed within 5 minutes |

[Table 1] (Continued)

| | | Comp. Ex. | Comp. Ex. | Comp. Ex. | Comp. Ex. | Comp. Ex. |
|---|---|---|---|---|---|---|
| | | Filler 13 | Filler 14 | Filler 15 | Filler 16 | Filler 17 |
| Core particle | | Fumed silica | Zirconia | Zirconia | Zirconia | Silica |
| Refractive index | | 1.46 | 1.50 | 1.51 | 1.48 | 1.51 |
| Zirconia primary particle diameter [nm] | 1 to 20 | - | 25 | 25 | 14 | - |
| Specific surface area [m$^2$/g] | 180 or less | 210 | 243 | 68 | 2 | 185 |
| Linseed oil absorption amount [g/100 g] | 200 or less | 231 | - | - | - | - |
| Linseed oil absorption amount [g/100 cm$^3$] | | 550 | - | - | - | - |
| Linseed oil absorption amount per surface area [g/m$^2$] | 0.02 or less | 0.0110 | - | - | - | - |
| Zirconia content | 4 to 60 | 0% | 18% | 18% | 18% | 18% |
| Transparency [%] | Higher than 20 | 100% | 9% | 7% | 15% | 63% |
| Polishability [s] (time until gloss develops) | | - | - | - | No gloss was observed within 5 minutes | 150 |

As is apparent from Table 1, the fillers 10 and 13 that show a linseed oil absorption amount exceeding 200 g per unit volume (100 cm$^3$) were found not to form a slurry even when mixed with the formulated resin at a 1:1 mass ratio, and to fail to exhibit sufficient fillability in the resin material. In addition, regarding the polishability of the fillers 9 and 12 that show a linseed oil absorption amount exceeding 0.02 g per unit surface area (m$^2$), the filler 9 was found to require 120 seconds which is a longer time than the other fillers before gloss develops, and the filler 12 was found not to develop any gloss as a result of polishing.

The fillers 1, 2, 3, 4, and 9 are fillers that were produced by substantially the same operations, except that the temperature in the calcination step was varied. Among these fillers, the specific surface area was found to decrease as the calcination temperature increased. The linseed oil absorption amount per unit surface area was found to increase as the specific surface area decreased, and the filler 9 that shows the linseed oil absorption amount exceeding 0.02 g/m$^2$ was found to exhibit a decrease in light transmittance and a decrease in polishability.

The fillers 14 and 15 are fillers that were produced by substantially the same operations as the fillers 1 and 2, except that the primary particles of zirconia in the aggregated particles have a size of 25 nm. The transparency was found to markedly decrease in a case where the primary particle size exceeded 20 nm.

The filler 16 includes particles that do not have a silica component as a second primary particle, and the filler 17 includes particles that do not have a zirconia component as a first primary particle, so that the transparency and the polishability were found to be markedly reduced.

**Claims**

1. A particulate material comprising an aggregate that is composed of primary particles having a volume-average particle diameter of 1 nm to 20 nm and including two or more metal elements, the aggregate having a surface composed of silica, wherein

    some of the primary particles each contain a heavy element having an atomic number of 38 or greater in the form of a metal oxide;
    at least some of the remaining primary particles are particles composed of silica;
    the particulate material has:
    a specific surface area of 180 m$^2$/g or less;
    a linseed oil absorption amount per unit volume (100 cm$^3$) of 200 g or less;
    a linseed oil absorption amount per unit surface area (m$^2$) of 0.02 g or less;
    a light transmittance of more than 20%; and
    a mass of the heavy element of 4% to 60% based on a total mass of the particulate material.

2. The particulate material according to claim 1, wherein

    a volume-average particle diameter is 0.1 μm to 10 μm, and
    the primary particles are bonded to each other by dehydration condensation or are fused to each other.

3. The particulate material according to claim 1, comprising an inorganic particulate material having a volume-average particle diameter of 1 nm to 200 nm and being dispersed into the primary particles.

4. The particulate material according to claim 3, wherein
the inorganic particulate material has a functional group represented by formula (1): -OSiX$^1$X$^2$X$^3$ and a functional group represented by formula (2): -OSiY$^1$Y$^2$Y$^3$ bonded to surfaces of silica particles (in the above formulas (1) and (2): X$^1$ is a phenyl group, a vinyl group, an epoxy group, a methacrylic group, an amino group, a ureido group, a mercapto group, an isocyanate group, or an acrylic group; X$^2$ and X$^3$ are each independently selected from - OSiR$_3$ and -oSiY$^4$Y$^5$Y$^6$; Y$^1$ is R; Y$^2$ and Y$^3$ are each independently selected from R and -oSiY$^4$Y$^5$Y$^6$; y4 is R; Y$^5$ and Y$^6$ are each independently selected from R and -OSiR$_3$; R is independently selected from alkyl groups having 1 to 3 carbon atoms; and any one of X$^2$, X$^3$, Y$^2$, Y$^3$, Y$^5$, and Y$^6$ may be bonded to any one of X$^2$, X$^3$, Y$^2$, Y$^3$, Y$^5$, and Y$^6$ of an adjacent functional group via -O-).

5. The particulate material according to claim 1, wherein the particulate material is dispersed in a transparent resin material and is used in a dental material.

6. The particulate material according to claim 5, wherein a refractive index of the particulate material differs from that of the transparent resin material by -0.01 to 0.01.

7. A dental material comprising:

the particulate material according to any one of claims 1 to 4; and
a transparent resin material in which the particulate material is dispersed, wherein
a content of the particulate material is 10% to 80% based on a total volume.

FIG.1

## INTERNATIONAL SEARCH REPORT

| International application No. |
|---|
| **PCT/JP2024/034288** |

| A. | CLASSIFICATION OF SUBJECT MATTER |
|---|---|

*A61K 6/76*(2020.01)i; *A61K 6/17*(2020.01)i; *C01B 33/18*(2006.01)i
FI:  A61K6/76; A61K6/17; C01B33/18 C

According to International Patent Classification (IPC) or to both national classification and IPC

| B. | FIELDS SEARCHED |
|---|---|

Minimum documentation searched (classification system followed by classification symbols)

A61K6/76; A61K6/17; C01B33/18

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS (STN)

| C. | DOCUMENTS CONSIDERED TO BE RELEVANT |
|---|---|

| Category* | Citation of document, with indication, where appropriate, of the relevant passages | Relevant to claim No. |
|---|---|---|
| X | JP 2001-302429 A (KABUSHIKI KAISHA SHOFU) 31 October 2001 (2001-10-31) claims 1-5, paragraphs [0001], [0019]-[0020], [0030]-[0034], [0036], [0043], [0047]-[0048], [0054]-[0055], example 1, table 1 | 1-7 |
| X | WO 2020/122192 A1 (KURARAY NORITAKE DENTAL INC.) 18 June 2020 (2020-06-18) claims 1, 13-17, 19, paragraphs [0001], [0023]-[0056], [0160], [0181], [0183], [0191]-[0194], production examples 1, 3, examples 1-13, 15, tables 2-4 | 1-7 |

☐ Further documents are listed in the continuation of Box C.  ☑ See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **11 October 2024** | **29 October 2024** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (July 2022)

**INTERNATIONAL SEARCH REPORT**
Information on patent family members

International application No.

**PCT/JP2024/034288**

| Patent document cited in search report | | | Publication date (day/month/year) | Patent family member(s) | Publication date (day/month/year) |
|---|---|---|---|---|---|
| JP | 2001-302429 | A | 31 October 2001 | US 2002/0022677 A1<br>claims 1-5, paragraphs [0001], [0023]-[0024], [0031]-[0035], [0037], [0044], [0048]-[0058], [0065], example 1, table 1<br>EP 1149573 A2 | |
| WO | 2020/122192 | A1 | 18 June 2020 | US 2022/0142874 A1<br>claims 1, 13-17, 19, paragraphs [0001], [0030]-[0065], [0195]-[0196], [0238]-[0241], [0245]-[0246], [0262], production examples 1, 3, examples 1-13, 15, tables 2-4<br>EP 3895682 A1 | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2021155399 A
- JP 2021172577 A